# EUROPEAN PATENT APPLICATION

(11) **EP 4 480 958 A1**
(43) Date of publication of application: **25.12.2024**
(21) Application number: 23756590.8
(22) Date of filing: 14.02.2023
(51) Int. Cl.: C07K 7/08, A61P 1/02, A61P 29/00, A61K 38/00, A61L 27/22, A61L 27/54

(54) **PEPTIDE HAVING ANTIBACTERIAL, ANTI-INFLAMMATORY OR TISSUE REGENERATION ABILITY AND USE THEREOF**

(30) Priority: 15.02.2022 KR 20220019713
(71) Applicant: Nibec Co., Ltd., Chungcheongbuk-do 27816 (KR); Seoul National University R&DB Foundation, Seoul 08826 (KR)
(72) Inventor: PARK, Yoon Jeong, Seoul 08291 (KR); CHUNG, Chong-Pyoung, Seoul 05618 (KR); LEE, Jue-Yeon, Gwacheon-si, Gyeonggi-do 13831 (KR); JO, Beom Soo, Seoul 05817 (KR)
(74) Representative: J A Kemp LLP
(86) International application number: PCT/KR2023/002114
(87) International publication number: WO 2023/158178

(57) **Abstract**

Disclosed are a peptide having antibacterial, anti-inflammatory, or tissue regeneration ability and use thereof. The peptide includes an amino acid sequence of SEQ ID NO: 1.

## Description

### [Technical Field]

This disclosure relates to a peptide having antibacterial, anti-inflammatory, or tissue regeneration ability and use thereof.

### [Background Art]

Many diseases and conditions are accompanied by inflammatory diseases as well as bacterial infections caused by various bacteria, etc. In particular, oral diseases are often accompanied by bacterial infections and inflammatory diseases. For example, in order for the treatment of periodontal diseases such as periimplantitis, periodontitis, and gingivitis to proceed normally, antibacterial and anti-inflammatory treatment should be carried out simultaneously. In addition to periodontal disease, damaged areas after tooth scaling or the condition of the tooth extraction socket after tooth extraction also require antibacterial and anti-inflammatory treatment. Therefore, there is a need for the development of substances that can be effectively applied to these diseases and conditions.

Additionally, about 700 types of microorganisms live in the oral cavity, and various types of bacteria attach to the surface of teeth to form biofilms, and these biofilms cause gingivitis, periodontitis, and periimplantitis. When a biofilm is formed, it is difficult for foreign substances to penetrate because the biofilm forms a physically thick layer, and because genetic mutations occur through interactions between cells within the biofilm, it is known that resistance to antibiotics is about 1,000 times higher than that of floating bacteria. Therefore, suppressing or removing biofilm formation will allow more effective prevention or treatment of gingivitis, periodontitis, or periimplantitis caused by bacterial infection.

Existing graft materials intended for tissue regeneration can only exert a regenerative function in areas without inflammation, and cannot perform a regenerative function if inflammation occurs after implantation. Therefore, if a substance with antibacterial and anti-inflammatory effects is applied together with a graft material, regeneration of damaged tissue can be achieved by suppressing inflammation that may occur in the early stages. For example, periodontal tissue damaged by periodontitis can easily achieve periodontal tissue regeneration effects if a graft material with antibacterial and anti-inflammatory effects is used in combination.

### [Disclosure]

### [Technical Problem]

The present disclosure provides a peptide with anti-inflammatory activity.

The present disclosure provides a peptide with antibacterial activity at the cellular level and in biofilms.

The present disclosure provides a peptide having cell penetrating ability.

The present disclosure provides a pharmaceutical composition including a peptide having anti-inflammatory or antibacterial activity.

The present disclosure provides a graft material including a peptide having anti-inflammatory or antibacterial activity.

### [Technical Solution]

The peptide according to embodiments is a peptide represented by the amino acid sequence of SEQ ID NO: 1.

The pharmaceutical composition according to embodiments includes the peptide represented by the amino acid sequence of SEQ ID NO: 1 as an active ingredient.

The graft material according to embodiments includes the peptide represented by the amino acid sequence of SEQ ID NO: 1 as an active ingredient.

Other embodiments of the present invention are included in the following detailed description.

### [Advantageous Effects]

A peptide according to an embodiment has anti-inflammatory or antibacterial activity and can be used for the treatment of inflammatory or bacterial diseases or conditions.

The peptide according to an embodiment has cell-penetrating ability, so that there is no need to attach a separate peptide or add other agents for the peptide to penetrate the cell. Additionally, it can assist cell penetration of other peptides through conjugation with other peptides that have low cell penetration ability.

The graft material according to the embodiment has anti-inflammatory or antibacterial activity, so that it can be effectively applied to periodontal disease areas such as periimplantitis and periodontitis, and can achieve a tissue regeneration effect.

### [Description of the Drawings]

FIG. 1 shows the results of analyzing the purity using liquid chromatography after synthesizing a peptide having the amino acid sequence of SEQ ID NO: 1.
FIG. 2 shows the results of analyzing the molecular weight using mass spectrum after synthesizing a peptide having the amino acid sequence of SEQ ID NO: 1.
FIG. 3 shows the results of enzyme-linked immunosorbent assay (ELISA) for IL-6 and TNF-α.
FIG. 4 shows the results of Western blot analysis of pNFkB and plkBa.
FIG. 5 shows the results of measuring the antibacterial activity of a peptide having the amino acid sequence of SEQ ID NO: 1.
FIG. 6 shows the results of treating a biofilm with a peptide having the amino acid sequence of SEQ ID NO: 1.
FIG. 7 shows the results of analyzing the degree of intracellular penetration of the peptide having the amino acid sequence of SEQ ID NO: 1 using a confocal microscope.
FIG. 8 shows the degree of intracellular penetration of a peptide having the amino acid sequence of SEQ ID NO: 1 into rabbit gingival tissue.
FIG. 9 shows the degree of intracellular penetration of a peptide having the amino acid sequence of SEQ ID NO: 1 into adult dog gingival tissue.
FIG. 10 shows the results of measuring the antibacterial activity of a collagen sponge block including a peptide having the amino acid sequence of SEQ ID NO: 1.
FIGS. 11 and 12 show the results of periodontal tissue regeneration confirmed by radiography by treating an adult dog periodontitis model with a collagen gel including a peptide having the amino acid sequence of SEQ ID NO: 1, and the degree of alveolar bone regeneration is numerically shown. The square represents the alveolar bone regenerated by the graft material.

### [Mode for Invention]

Hereinafter, the embodiments will be described in detail so that those skilled in the art can easily perform the embodiments. The embodiments may be implemented in various different forms, and the present disclosure is not limited only to the specific embodiments described herein.

Unless the definition of some terms used in the present disclosure is defined otherwise below, all technical and scientific terms used herein have the same meaning as commonly understood by one of ordinary skill in the art to which the present invention belongs.

The techniques and processes described in this disclosure are generally performed according to conventional methods, which are presented throughout this application. In general, nomenclatures and experimental procedures in molecular biology, biochemistry, analytical chemistry, and cell culture used in this disclosure are well known in the art and are the same as those commonly used.

In one aspect, the present disclosure relates to a peptide having anti-inflammatory or antibacterial activity. The peptide is a peptide having the amino acid sequence of SEQ ID NO: 1.

### SEQ ID NO: 1: GKCSTRGRKSSRRKK

In an aspect, after treatment with the peptide having the amino acid sequence of SEQ ID NO: 1, the intracellular release amount of inflammatory markers IL-6 and TNF-α was measured using enzyme-linked immunosorbent assay (ELISA), and the peptide having the amino acid sequence of SEQ ID NO: 1 exhibited anti-inflammatory activity almost similar to that of IL-4, a cytokine with anti-inflammatory effect. In addition, the inhibitory effect on the expression of pNF-κB, an inflammation-related enzyme, by the peptide having the amino acid sequence of SEQ ID NO: 1 was confirmed by Western blot. As a result, the peptide having the amino acid sequence of SEQ ID NO: 1 inhibits the expression of pNF-κB induced by LPS.

In another aspect, it was confirmed that the peptide having the amino acid sequence of SEQ ID NO: 1 has excellent antibacterial activity against periodontitis-causing bacteria, Aggregatibacter actinomycetemcomitans and Porphyromonas gingivalis, and has equal or similar antibacterial activity compared to minocycline, which is widely used in the treatment of periodontitis. In addition, as a result of treating an artificial biofilm made using periodontitis-causing bacteria with a peptide having the amino acid sequence of SEQ ID NO: 1, it was confirmed that it had antibacterial activity and further cell penetrating ability.

From another viewpoint, the present disclosure relates to a peptide having cell penetrating ability.

In one aspect, it was confirmed through in vitro and in vivo experiments that a peptide having the amino acid sequence of SEQ ID NO: 1 penetrates into cells. Specifically, as a result of treating mouse macrophages (RAW264.7) with a peptide having the amino acid sequence of SEQ ID NO: 1 and confirming whether it penetrates into the cells, it was confirmed that the peptide having the amino acid sequence of SEQ ID NO: 1 penetrated into the cells. In addition, it was confirmed that the peptide having the amino acid sequence of SEQ ID NO: 1 penetrates not only at the cell level but also at the tissue level. When the gingival tissue of a living rabbit and an adult dog were treated with a peptide having the amino acid sequence of SEQ ID NO: 1, it was confirmed that it penetrated into the gingival tissue of a living rabbit and the gingival tissue of an adult dog.

In the case of periodontitis or periimplantitis, various types of periodontitis-causing bacteria cluster together to form a thick biofilm. Because these biofilms become increasingly resistant to antibiotics, they are difficult to remove by administering antibiotics alone. Therefore, it is necessary to pass through this biofilm and at the same time have antibacterial activity against periodontitis-causing bacteria that make up the biofilm. Since the peptide having the amino acid sequence of SEQ ID NO: 1 can penetrate into cells on its own, there is no need to use an additional carrier. Therefore, it can penetrate the biofilm formed on teeth to exhibit antibacterial activity or easily penetrate gingival tissue. Furthermore, by using this cell/tissue penetrating ability, the desired anti-inflammatory effect can be increased by chemically conjugating the peptide with an anti-inflammatory function without cell penetrating ability to impart cell penetrating ability.

From another viewpoint, the present disclosure relates to a pharmaceutical composition including a peptide having the amino acid sequence of SEQ ID NO: 1 as an active ingredient.

The pharmaceutical composition can be effectively applied for treating dental infectious diseases, and the dental infectious diseases may be selected periodontitis, gingivitis, and peri-implantitis.

The composition for treating dental infectious diseases may include 10⁻³ to 10 parts by weight of the peptide, and more preferably 10⁻² to 10⁻¹ parts by weight, based on 100 parts by weight of the composition for treating dental infectious diseases. This is because if the peptide is included in less than 10⁻³ parts by weight, the antibacterial or anti-inflammatory effect is not significant, and if it is included in more than 10 parts by weight, the effect does not increase proportionally, so that there is no practical benefit from increasing the peptide content.

The composition for treating dental infectious diseases may additionally include one or more selected from propolis, xylitol, and proteolytic enzymes. The propolis or xylitol can be included to improve sensory characteristics, and proteolytic enzymes can be added to increase the antibacterial effect and lipopolysaccharide removal effect.

Pharmaceutically acceptable carriers for composition for treating dental infectious diseases may include excipients such as starch, lactose, calcium carbonate, calcium phosphate, etc., conjugates such as starch, gum arabic, carboxymethyl cellulose, hydroxymethyl cellulose, and crystalline cellulose, lubricants such as magnesium stearate, talc, etc., disintegrants such as carboxymethyl cellulose calcium, talc synthetic aluminum silicate, etc., diluents such as water, vegetable oil, etc., and a mixture thereof.

The formulation of the composition for treating dental infectious diseases is not particularly limited, but may be formulated as powder, fine granules, liquid, powder, spray, ointment, and gel formulation, and is most preferably formulated as a gel formulation.

In addition, formulations in which the peptide of the present invention is mixed into a composition for treating inflammation are formulated using conventional ingredients and methods, such as lotions, creams, ointments, emulsions, foundations, oils, packs, soaps including medicated soaps, body soap, lip rouge, nail cosmetics, eye cosmetics, perfume, face wash, oral care products such as toothpaste, mouthwash, etc., deodorants, bath solvents, shampoo, conditioner, hair toy, hair spray, and hair dye. In addition, the various compositions including the ingredients according to the present invention can be arbitrarily used in the form of solution, cream, paste, gel, gel, foam, solid, and powder.

From another perspective, the present disclosure relates to a graft material including a peptide having the amino acid sequence of SEQ ID NO: 1 as an active ingredient. The graft material can include graft materials from various fields such as a skin graft material, a bone graft material, a musculoskeletal regeneration material, a nerve regeneration material, and a vascular regeneration material. The peptide having the amino acid sequence of SEQ ID NO: 1 can be used in combination with various graft matrices such as extracellular matrix proteins, bone minerals, chitosan, biodegradable synthetic polymers, etc.

The extracellular matrix protein may be one or more selected from collagen, hyaluronic acid, elastin, chondroitin sulfate, and fibroin. These extracellular matrices can be made from both human and animal derived proteins and recombinant proteins produced from microorganisms. Among extracellular matrix proteins, collagen may be the most suitable. Collagen is produced by fibroblasts in the human body and is a main component of connective tissue, which is most commonly found in the body, and is a fibrous protein distributed throughout our body, including bones, skin, joints, membranes of various organs, and hair. In addition, a lot of collagen exists around the axons of central and peripheral nerves. Since collagen hydrogels present in the body have slightly different functions and roles depending on the tissue area, the collagen concentration and degree of orientation of collagen hydrogels in each area are different. For example, bones are characterized by a structure in which micro/nanocrystalline minerals are located between oriented collagen fibers. Collagen is an absorbable material in the body that decomposes easily, so when implanted into the body, it is suitable as a graft matrix because it can gradually decompose and be replaced by regenerated bone. In the case of collagen, it is preferable to use type 1 or type 3 isolated from pig skin.

The bone mineral component may include one or more selected from bio-derived bone mineral powder derived from allogeneic or xenogeneic bone, synthetic hydroxyapatite, and calcium phosphate cement (CPC) powder including tetracalcium phosphate (TTCP), dicalcium phosphate anhydride (DCPA), and monocalcium phosphate monohydrate (MCPM).

The biodegradable synthetic polymer may be one or more selected from polyglycolide, polylactide, polyglycolide lactide, and polycatrolactone.

The graft matrix may be in a form of a preparation consisting of paste, powder, putty, gel, hydrogel, matrix, granule, particle, lyophilized powder, lyophilized bone, demineralized lyophilized bone, fresh or fresh frozen bone, cortical cancellous bone mix, pellet, strip, plug, membrane, lyophilized powder that is returned to water to form a wet cake, sphere, sponge, block, morcelle, stick, wedge, cement, or amorphous particles.

In one aspect, the graft material may exist as a combination of a first agent including collagen as a main ingredient and a second agent including a peptide having the amino acid sequence of SEQ ID NO: 1 formulated to allow immersion of the first agent, and these are mixed and used before implantation.

The first agent including collagen as a main ingredient may include a collagen sponge block with a size of 6X5X7 mm or 8X7X9 mm and may include collagen and bone mineral complex with a size of 6X6X3 mm, 6X6X6 mm, 7X8X9 mm, or 9X10X11 mm. The first and second agents may be mixed and used before implantation. The advantage of mixing and using them right before implantation is that collagen and peptides can be kept in a sterile state.

For a total of 100 parts by weight of the graft material, 10⁻⁴ to 0.5 parts by weight of the peptide may be contained, and more preferably 10⁻³ to 10⁻¹ parts by weight. This is because if the peptide is included in less than 10⁻³ parts by weight, the regenerative effect is not significant, and if it is contained in more than 0.5 parts by weight, it is difficult to include it in a graft material.

Hereinafter, preferred experimental examples are presented to aid understanding of the present invention. However, the following experimental examples are merely illustrative of the present invention and the scope of the present invention is not limited to the following experimental examples.

### Peptide synthesis

The peptide of SEQ ID NO: 1 was synthesized from the C terminus using a synthesis device using the F-moc solid phase chemical synthesis method. That is, it was synthesized using Rink resin (0.075 mmol/g, 100 to 200 mesh, 1% DVB crosslinking) with Fmoc-(9-Fluorenylmethoxycarbonyl) bound as a blocking group, and after adding 50 mg of Rink Amide MBHA resin to the synthesizer, the resin was swelled with DMF, and then a 20% piperidine/DMF solution was used to remove the Fmoc-group. In order from the C terminus, 0.5 M amino acid solution (solvent: DMF), 1.0 M DIPEA (solvent: DMF&NMP), and 0.5M HBTU (solvent: DMF) were added in 5, 10, and 5 equivalents, respectively and reacted for 1 to 2 hours under a nitrogen stream. After each deprotection and coupling step, washing was performed twice with DMF and methanol. Even after coupling the last amino acid, deprotection was performed to remove the Fmoc-group.

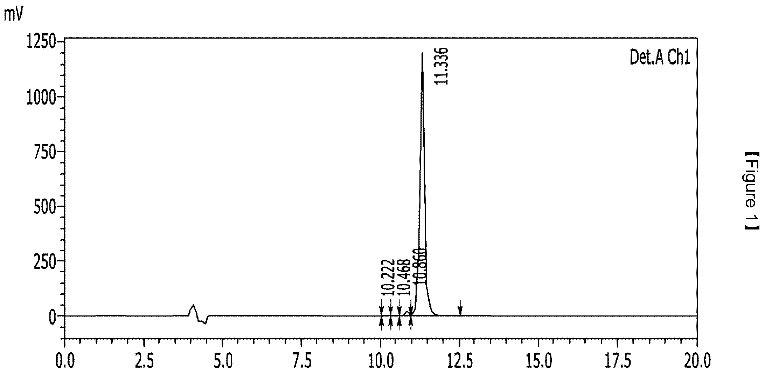

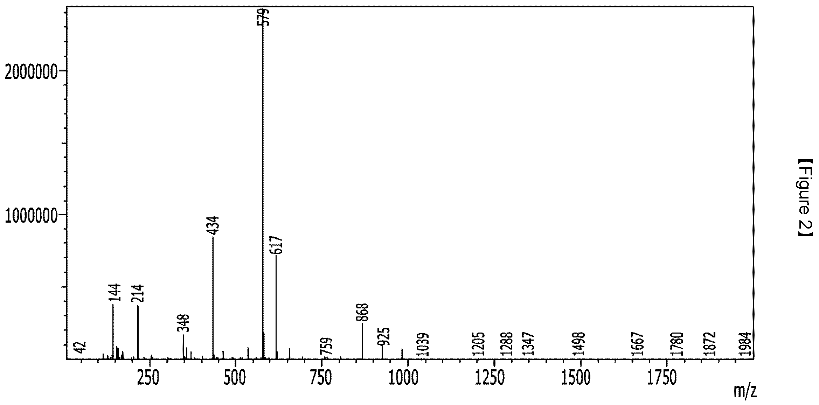

The ninhydrin test method was used to confirm the synthesis. After the test and the completed resin was dried, Reagent K cleavage cocktail was added at a ratio of 20 ml per 1g of resin, and shaken for 3 hours, and the cocktail including dissolved resin and peptide was separated through filtering. Cold ether was added to the filtered solution to crystallize the peptide into a solid phase, which was separated by centrifugation. At this time, the Reagent K cleavage cocktail was completely removed through several washings with ether and centrifugation. The crude obtained in this way was dissolved in distilled water and separated and purified using liquid chromatography. The purified peptide was lyophilized. The results of analyzing the purity and measuring the mass spectrum by liquid chromatography are illustrated in FIGS. 1 and 2.

Referring to FIGS. 1 and 2, it can be confirmed that the synthesized peptide has a purity of 98.2%, a molecular weight of 1733.99, and a 15-mer amino acid sequence.

### Anti-inflammatory Activity Evaluation

### 1) Measurement of production of inflammatory cytokines (IL-6 and TNF-α)

Anti-inflammatory activity was verified using human monocyte cell lines (THP-1, ATCC, TIB-202). In the case of monocyte cell lines, PMA (Phorbol 12-myristate 13-acetate, Merck, P1585) was inoculated to differentiate monocytes into macrophages, and then treated with 1 µg/ml LPS (lipopolysaccharide, Merck, L3024), an inflammation inducer. 16 hours later, 200 µM of the peptide having the amino acid of SEQ ID NO: 1 and 20 ng/ml of IL-4 (R&D system, 204-IL-010/CF) as a positive control were inoculated, and 24 hours later, the culture medium of the cells was obtained. The amount of pro-inflammatory cytokines IL-6 (R&D system, D6050) and TNF-α secreted by cells present in the medium was confirmed through ELISA (enzyme-linked immunosorbent assay).

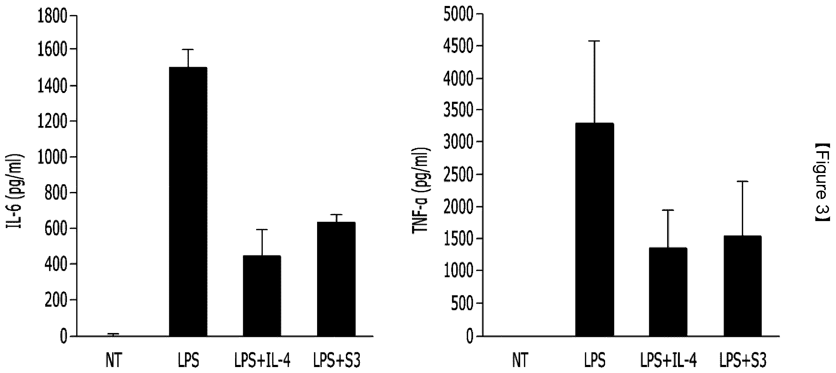

As a result, as shown in FIG. 3, when treated with the peptide having the amino acid sequence of SEQ ID NO: 1 (LPS+S3), it was confirmed that IL-6 and TNF-α values were lowered to a similar level as when treated with IL-4. When treated with LPS alone, IL-6 and TNF-α values increased. Therefore, it was found that the peptide having the amino acid sequence of SEQ ID NO: 1 has an anti-inflammatory effect.

### 2) Western blot of pNFkB and plkBa

Raw264.7 cells were purchased from ATCC (American Type Culture Collection) and used in the experiment. Raw264.7 cells were cultured in a CO₂ incubator at 37 °C supplied with 5% CO₂ and 95% air using DMEM (Dulbecco's modified Eagle medium) medium supplemented with 10% FBS and 100 units/ml antibiotics (penicillin-streptomycin).

Raw264.7 cells were treated with 200 µM of the peptide having the amino acid sequence of SEQ ID NO: 1 for 30 minutes, and then treated with 1 µg/ml LPS (lipopolysaccharide, Merck, L3024). After 8 hours, the treated cells were collected, protease inhibitor cocktail and phosphatase inhibitor cocktail were added to Radioimmunoprecipitation assay buffer (RIPA buffer solution, Thermo Fisher, 89900), and the cells were treated to lyse the cells. After centrifuging the cell lysate and collecting the supernatant, the concentration of the cell lysate was measured using a bicinchoninic acid assay (BCA assay, Thermo Fisher, 23227), and RIPA buffer solution was added thereto to prepare the cell lysate to a final concentration of 5 mg/mL. Then, it was mixed with a buffer solution including 1M Tris-HCl (pH 6.8), 50% glycerol, and 10% SDS in a 4:1 ratio, and then sodium dodecyl sulfate-poly acrylamide gel electrophoresis (SDS-PAGE) was performed. Then, the polyacrylamide gel was transferred to a nitrocellulose membrane, the membrane was blocked with a blocking solution (5% skim milk in Tris-buffered saline with 0.1% Tween^{®}20), and then primary antibodies (phosphorylated NF-κB (Signaling Technology, 3033), NF-κB (Signaling Technology, 8242), phosphorylated IκBα (Cell Signaling Technology, 9246), IκBα (Cell Signaling Technology, 4812) and β-actin (Santa Cruz Biotechnology, sc-47778)) were reacted with the blocking solution at a 1:1000 ratio in the refrigerator overnight. Then, the secondary antibody for each primary antibody was reacted with blocking solution 1:2000 at room temperature for 1 hour, then reacted with ECL (enhanced chemiluminescence, Thermo Fisher, 34094) solution, and then film was developed using ChemiDoc Imaging Systems (Bio-Rad). Phosphorylated NF-κB and phosphorylated plkBa are indicators that can confirm a degree of inflammatory response at the cellular level. The higher the inflammatory response, the more phosphorylated NF-κB and phosphorylated plkBa expression can be confirmed.

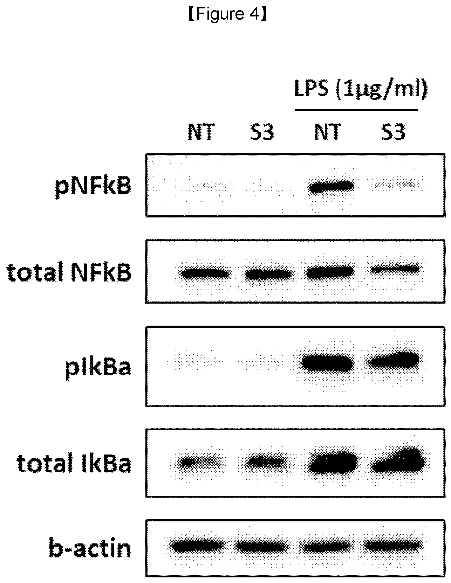

As a result, as shown in FIG. 4, when only LPS was treated, the expression of pNFκB and plkBa increased, but it was confirmed that when treated with LPS after treatment with the peptide having the amino acid sequence of SEQ ID NO: 1, the expression of pNFκB and plkBa was suppressed. Therefore, it was found that the peptide having the amino acid sequence of SEQ ID NO: 1 has an anti-inflammatory effect.

However, when only the peptide having the amino acid sequence of SEQ ID NO: 1 was treated, there was no effect on the expression of pNFκB and plkBa in cells, which indirectly proves that the peptide is safe.

### Antibacterial activity evaluation

### 1) Inhibiting growth of periodontitis-causing bacteria

To evaluate the antibacterial activity, representative periodontitis-causing bacteria, Aggregatibacter actinomycetemcomitans and Porphyromonas gingivalis, were used. Each bacterium was inoculated into BHI (brain heart infusion) liquid medium and cultured in an anaerobic incubator at 37 °C and 120 rpm for 48 hours. After incubation, the peptide having the amino acid sequence of SEQ ID NO: 1 was treated at 100 µM, 250 µM, and 500 µM. As a positive control, minocycline (Merck, M9511) was inoculated at 500 ng/ml, and then 24 hours later, the absorbance was measured at 600 nm using a spectrophotometer to confirm a degree of bacterial growth.

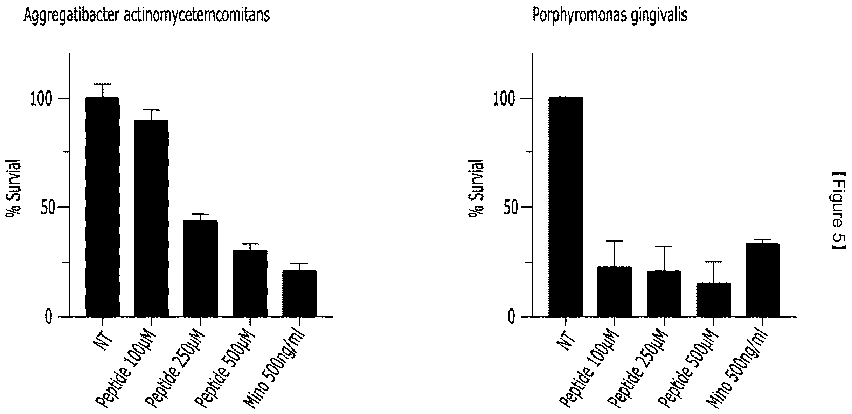

As a result, as shown in FIG. 5, when treated with a peptide having the amino acid sequence of SEQ ID NO: 1, the survival rate of bacteria was less than or equal to 50% for Aa (Aggregatibacter actinomycetemcomitans) at 250 µM or more, and the survival rate of bacteria was found to be less than or equal to 50% for Pg (Porphyromonas gingivalis) at 100 µM or more.

Meanwhile, when compared to the effect of minocycline, when treated with Aa at a concentration of 500 µM, the survival rate was higher than when treated with minocycline 500 ng/ml, but showed a similar effect. The minocycline only has antibacterial activity, whereas the peptide having the amino acid sequence of SEQ ID NO: 1 has both antibacterial activity and anti-inflammatory activity, so that even if there is a slight difference in antibacterial activity, this can be compensated for. For Pg, it showed a better effect than minocycline in all concentration ranges.

### 2) Evaluation of antibacterial activity in biofilm

Periodontitis-causing bacteria exist in the oral cavity in a form of a biofilm, and thus in order to imitate this, an artificial biofilm was created on the surface of a titanium disk using A. a. (Aggregatibacter actinomycetemcomitans) and P. g. (Porphyromonas gingivalis) bacteria. Saliva and bacteria were mixed on a titanium disk, placed in BHI liquid medium, and cultured in an anaerobic incubator at 37 °C. After 72 hours, 50 µg/ml (=29 µM), 100 µg/ml (=58 µM), 250 µg/ml (=144 µM), and 500 µg/ml (=288 µM) peptides and 1 µg/ml minocycline as a positive control were inoculated. 24 hours later after inoculation, a titanium disk with biofilm formed on the surface was obtained and the biofilm was stained using the Filmtracer LIVE/DEAD Biofilm Viability Kit (Thermo Fisher, L10316). Live bacteria are stained green, and dead bacteria are stained red. The titanium disk was observed using a confocal scanning fluorescence microscope.

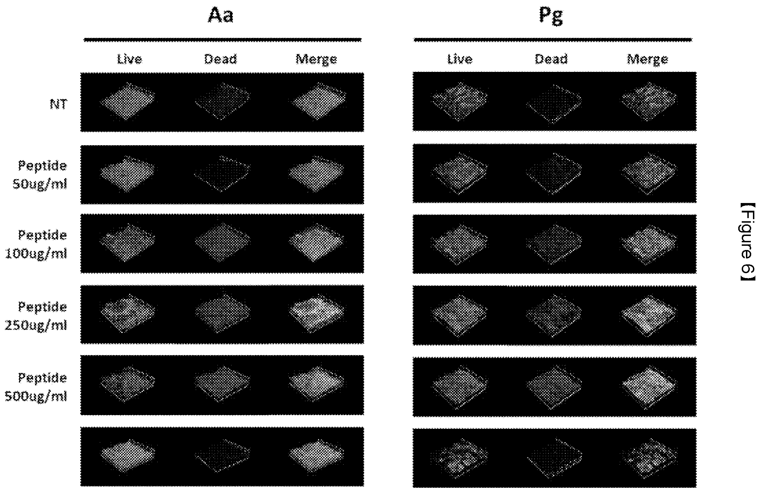

As a result, as shown in FIG. 6, minocycline used as a positive control was unable to penetrate the biofilm and kill the bacteria, so that almost no killed bacteria (red) were observed. On the other hand, when treated with a peptide having the amino acid sequence of SEQ ID NO: 1, killed bacteria (red) were observed starting at a concentration of 50 µg/ml (=29 µM). Therefore, in the case of bacteria existing in the form of a biofilm, existing antibiotics cannot kill them, but the peptide having the amino acid of SEQ ID NO: 1 penetrates the biofilm and has antibacterial activity.

### Cell/tissue penetrating ability evaluation

### 1) Evaluation of cell penetration ability at the cellular level

The N-terminus of the peptide having the amino acid of SEQ ID NO: 1 prepared in Example 1 was fluorescently labeled with Rhodamine B (Tokyo chemical industry Co., LTD., A5102). Raw264.7 cells were distributed 3×10⁴ each into a 4-well chamber and cultured in DMEM medium for 24 hours to stabilize the cells. After 24 hours, fluorescently labeled peptide was added to the medium at concentrations of 50, 100, and 200 µM. After 1 hour of peptide treatment, a degree of penetration of the peptide into cells was observed using a confocal scanning fluorescence microscope. Cell nuclei were stained using 4',6-diamidino-2-phenylindole (DAPI, Thermo Fisher, D1306). DIC represents the case observed under a general microscope, Nuceli represents the nucleus of a cell, Peptide represents the degree of penetration of a peptide bound to rhodamine B, and MERGE represents an image that overlaps images of DIC, Nuclei, and the penetrated peptide.

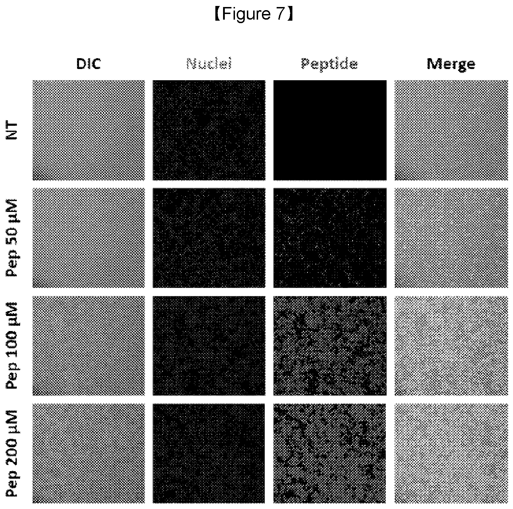

As a result, as shown in FIG. 7, fluorescence of rhodamine B (red) could be confirmed in cells treated with the peptide having the amino acid of SEQ ID NO: 1, but red color could not be confirmed in the untreated experimental group. In addition, it was found that as the concentration of peptide increased, the intensity of fluorescence also increased.

### 2) Evaluation of tissue penetration ability in animal tissue

The amine group of the peptide having the amino acid of SEQ ID NO: 1 was labeled with fluorescence using Alexa Fluor^{™} 680 NHS Ester (Thermo Fisher, A20008). The fluorescent labeling method followed the method provided by the manufacturer. The fluorescently labeled peptide was dissolved in water for injection at a concentration of 250 mg/ml, and 10 µL was injected into the gingival sulcus of the premolars of an adult dog (beagle dog) and the anterior gingival sulcus of a rabbit. After 2 hours, to check the permeability of the peptide in the tissue, the animal was sacrificed, the gingival tissue was collected, and tissues were embedded using Tissue-Tek^{®} O.C.T. Compound (Sakura Finetek, 4583). After sectioning the embedded tissue at 10 µm thickness, fluorescently labeled peptides were confirmed using a confocal scanning fluorescence microscope. The nuclei of tissue cells were stained using DAPI.

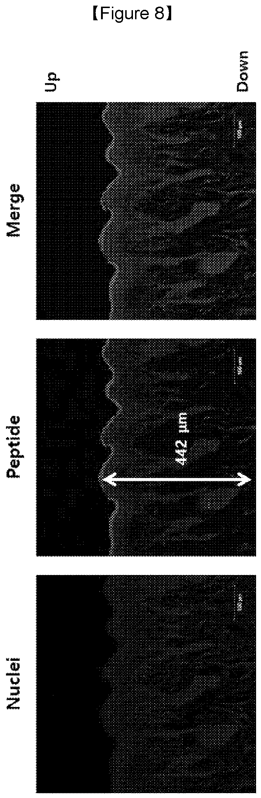

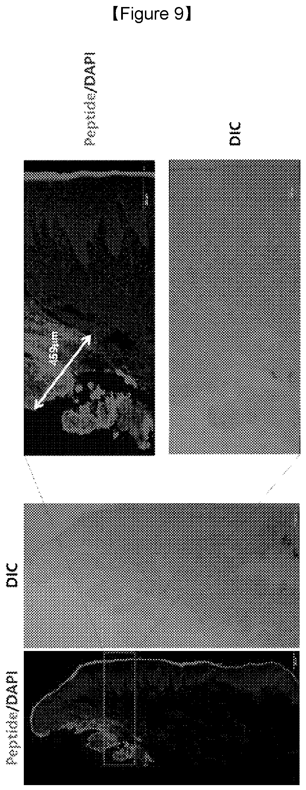

As a result, it was confirmed that the fluorescently labeled peptide penetrated the gingival tissue cells and reached about 500 (440 to 459) µm from the results of the gingival tissue of a rabbit in FIG. 8 and the gingival tissue of an adult dog in FIG. 9.

Therefore, it was confirmed that the peptide having the amino acid sequence of SEQ ID NO: 1 penetrates not only the cell level but also tissue.

### Evaluation of antibacterial activity of graft materials

500 µg/0.5 ml of the peptide having the amino acid of SEQ ID NO: 1 was immersed in the collagen sponge block with a size of 6X5X7mm, left at 4 °C for 8 hours, and then lyophilized.

As a positive control, 250 ng/0.5ml of minocycline (Merck, M9511) was immersed in a collagen sponge block with a size of 6X5X7mm, left at 4 °C for 8 hours, and then lyophilized. In order to evaluate the antibacterial activity of a graft material including the peptide having the amino acid of SEQ ID NO: 1, representative periodontitis-causing bacteria, Aggregatibacter actinomycetemcomitans and Porphyromonas gingivalis, were used. Each bacterium was inoculated into BHI (brain heart infusion) liquid medium and cultured in an anaerobic incubator at 37 °C and 120 rpm for 48 hours. After culturing, a collagen sponge block including the peptide having the amino acid sequence of SEQ ID NO: 1 of Example 2 was added to the medium. After 24 hours, the absorbance was measured at a wavelength of 600 nm to confirm a degree of bacterial growth.

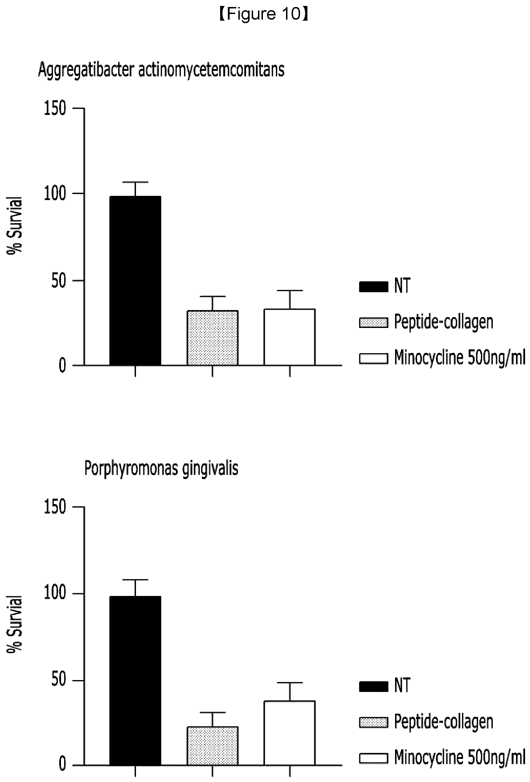

As a result, as shown in FIG. 10, when the collagen sponge block including the peptide having the amino acid sequence of SEQ ID NO: 1 was treated, the survival rate of Aa (Aggregatibacter actinomycetemcomitans) and Pg (Porphyromonas gingivalis) was less than or equal to 40%. Therefore, it was proven that an antibacterial effect exists even when the peptide having the amino acid sequence of SEQ ID NO: 1 was applied to the collagen sponge block.

### Evaluation of periodontal tissue regeneration ability of graft material

Collagen gel was dissolved in 0.1 M PBS at a concentration of 4%. 125 mg of a peptide having the amino acid of SEQ ID NO: 1 was dissolved in 1 g of collagen gel. As a positive control, 20 mg of minocycline was dissolved in 1 g of the same collagen gel.

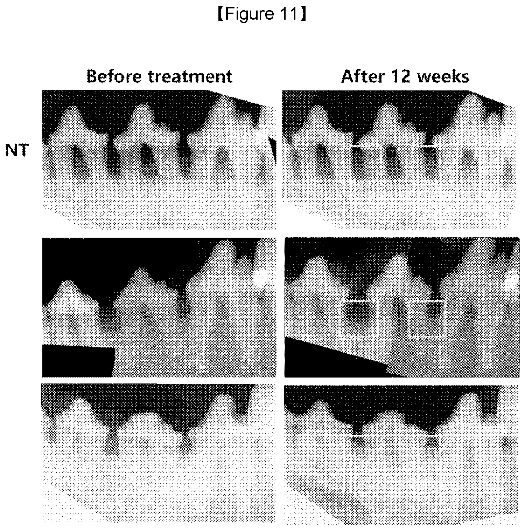

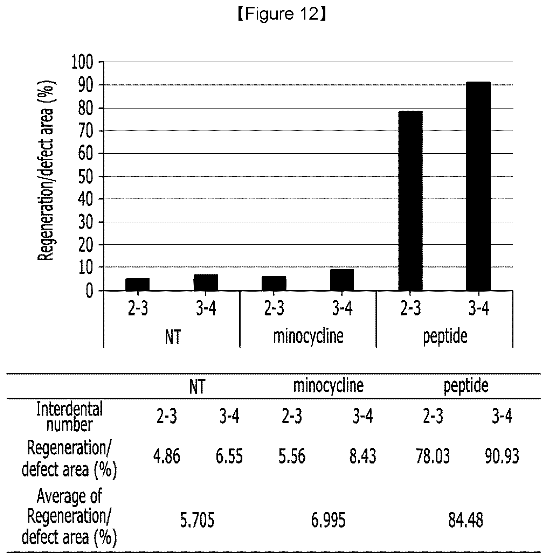

To evaluate the periodontal tissue regeneration ability of a graft material including a peptide having the amino acid of SEQ ID NO: 1, a collagen sponge block including the peptide was implanted after inducing periodontitis in a beagle dog. Periodontitis was induced by wrapping multiple layers of dental wire around the cervical region of the 2nd, 3rd, and 4th mandibular premolars. Three months after wrapping the wire, the wire was removed, and scaling and root planning were performed. 15 µl of collagen gel including the peptide having the amino acid of SEQ ID NO: 1 and collagen gel including minocycline were each applied to the gingival sulcus twice a week for 5 weeks. Periodontal condition was observed for 12 weeks, radiography (X-ray) was taken, and alveolar bone regeneration was observed. As a result, as shown in FIGS. 11 and 12, the group to which nothing was applied (NT) did not produce alveolar bone after 12 weeks. The group implanted with collagen gel including minocycline showed a similar degree of alveolar bone regeneration as the NT group. However, when the collagen gel applied with the peptide having the amino acid of SEQ ID NO: 1 was implanted, the regeneration of the alveolar bone (white part in the square) increased by more than 10 times compared to the group implanted with the collagen gel including minocycline. Therefore, the collagen gel to which the peptide having the amino acid of SEQ ID NO: 1 was applied was proven to have an excellent periodontal tissue regeneration effect.

Although the experimental examples were described above, the scope of rights is not limited thereto. The embodied forms can be implemented with various modifications within the scope of the detailed description of the invention and the attached drawings, and this naturally also falls within the scope of rights.

### [Industrial Applicability]

It can be used as a pharmaceutical composition for the treatment of inflammatory or bacterial diseases or conditions. It can be used as a pharmaceutical composition to treat periodontal diseases such as periimplantitis and periodontitis.

### [Sequence Listing Text]

SEQ ID NO: 1: GKCSTRGRKS SRRKK

## Claims

1. A peptide with anti-inflammatory activity consisting of the amino acid sequence of SEQ ID NO: 1.

2. An anti-inflammatory pharmaceutical composition comprising the peptide of claim 1 as an active ingredient.

3. The anti-inflammatory pharmaceutical composition of claim 2, wherein
the anti-inflammatory pharmaceutical composition is for treating dental infectious diseases.

4. A peptide with antibacterial activity against bacteria and biofilms consisting of the amino acid sequence of SEQ ID NO: 1.

5. An antibacterial pharmaceutical composition comprising the peptide of claim 4 as an active ingredient.

6. The antibacterial pharmaceutical composition of claim 5, wherein
the antibacterial pharmaceutical composition is for treating dental infectious diseases.

7. A peptide with anti-inflammatory and antibacterial properties consisting of the amino acid sequence of SEQ ID NO: 1.

8. An anti-inflammatory and antibacterial pharmaceutical composition comprising the peptide of claim 7 as an active ingredient.

9. The anti-inflammatory and antibacterial pharmaceutical composition of claim 5, wherein
the anti-inflammatory and antibacterial pharmaceutical composition is for treating dental infectious diseases.

10. A peptide with cell-penetrating and tissue-penetrating abilities consisting of the amino acid sequence of SEQ ID NO: 1.

11. A pharmaceutical composition comprising a peptide consisting of the amino acid sequence of SEQ ID NO: 1 as an active ingredient.

12. The pharmaceutical composition of claim 11, wherein
the pharmaceutical composition is for treating dental infectious diseases.

13. The pharmaceutical composition of claim 12, wherein
the dental infectious diseases is gingivitis, periodontitis, or periimplantitis.

14. A graft material comprising a peptide consisting of the amino acid sequence of SEQ ID NO: 1 as an active ingredient.

15. The graft material of claim 14, wherein
the graft material includes extracellular matrix protein, bone mineral, chitosan, or biodegradable synthetic polymer.

16. The graft material of claim 14, wherein
the graft material includes 10⁻⁴ to 0.5 parts by weight of a peptide consisting of the amino acid sequence of SEQ ID NO: 1.

17. The graft material of claim 14, wherein
the graft material has antibiotic, anti-inflammatory, or periodontal tissue regeneration abilities.
